(19) Europäisches Patentamt
European Patent Office
Office européen des brevets

(11) Veröffentlichungsnummer: **0 220 539**
**B1**

(12) # EUROPÄISCHE PATENTSCHRIFT

(45) Veröffentlichungstag der Patentschrift: **22.11.90**

(51) Int. Cl.$^5$: **C07D 285/10,** A61K 31/41

(21) Anmeldenummer: **86113716.4**

(22) Anmeldetag: **03.10.86**

(54) **Neue Kristallmodifikation des 3-Methoxy-4-(4'-aminobenzolsufonamido)-1,2,5-thiadiazols, Verfahren zu deren Herstellung und diese enthaltende pharmazeutische Zubereitungen.**

(30) Priorität: **04.10.85 DE 3535502**

(43) Veröffentlichungstag der Anmeldung: **06.05.87 Patentblatt 87/19**

(45) Bekanntmachung des Hinweises auf die Patenterteilung: **22.11.90 Patentblatt 90/47**

(84) Benannte Vertragsstaaten: **AT BE CH DE ES FR GB GR IT LI LU NL SE**

(56) Entgegenhaltungen:
**DE-C-1 175 683**
**DE-C-2 701 632**

**CHEMICAL ABSTRACTS, Babd 96, Nr. 24, 14. Juni 1982, Columbus, Ohio, USA KOO, CH.H.; CHUNG, Y.J.; SHIN, H.S.; SUH, J.S. "Crystals and molecular structures of sulfametrole" Seite 631, Spalten 1, 2, Zusammenfassung-Nr. 208 771s**

**Die Akte enthält technische Angaben, die nach dem Eingang der Anmeldung eingereicht wurden und die nicht in dieser Patentschrift enthalten sind.**

(73) Patentinhaber: **Hafslund Nycomed Pharma Aktiengesellschaft, St. Peter-Strasse 25, A-4021 Linz(AT)**

(84) Benannte Vertragsstaaten: **BE CH DE ES FR GB GR IT LI LU NL SE AT**

(72) Erfinder: **Burger, Artur, Mag, Dr., Mariahilfpark 3, A-6020 Innsbruck(AT)**
Erfinder: **Bolitschek-Dialer, Regine, Mag. Dr., Panholzerweg 13, A-4033 Linz(AT)**
Erfinder: **Reithmayr, Karl, Mag. Dr., Karl Rennerstrasse 4, A-4040 Linz(AT)**
Erfinder: **Weidinger, Ernst, Mag., Dinghoferstrasse 63, A-4020 Linz(AT)**

(74) Vertreter: **Kunz, Ekkehard, Dr., Chemie Holding AG Patentwesen St. Peter-Strasse 25, A-4021 Linz(AT)**

EP 0 220 539 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft das neue kristalline Halbhydrat des 3-Methoxy-4-(4′-aminobenzolsulfonamido)-1,2,5-thiadiazols, ein Verfahren zu dessen Herstellung und pharmazeutische Zubereitungen, die dieses Halbhydrat als Wirkstoff enthalten.

3-Methoxy-4-(4′-aminobenzolsulfonamido)-1,2,5-thiadiazol (Sulfametrol) ist ein Chemotherapeutikum mit ausgeprägten antibakteriellen Eigenschaften, welches allein oder in Kombination mit 2,4-Diamino-5-(3,4,5-trimethoxybenzyl)-pyrimidin (Trimethoprim) gegenüber einem breiten Spektrum von bakteriellen Erregern gute Wirksamkeit zeigt.

Sulfametrol und dessen Herstellung ist in den DE-PSen-1 175 683 und 2 701 632 beschrieben. Bakteriologische Untersuchungen zur therapeutischen Wirksamkeit von Sulfametrol und dessen Kombination mit Trimethoprim wurden in der Fachliteratur mehrmals veröffentlicht (vg. dazu G. Nabert-Bock et al. in Arzneimittel-Forsch. 27, 1109, 1977 und S. Bleyer et al. in Wiener Med. Wochenzeitschriften 130, 448, 1980).

Eine wasserfreie Form von Sulfametrol ist die einzige Kristallmodifikation, die bisher bekannt ist (vgl. C.H. Koo et al., Bull. Korean Chem. Soc. 3,9, 1982; C.A. 96, 208 771, 1982) und die für die Herstellung von festen und flüssigen Arzneiformen bisher verwendet wurde. Diese wasserfreie Kristallmodifikation kann jedoch bei der Herstellung verschiedener Arzneiformen nicht restlos befriedigen. So wird beispielsweise bei Suspensionen für die orale Applikation, die Sulfametrol in der bekannten Kristallmodifikation allein oder in Kombination mit Trimethoprim enthalten, unter ungünstigen Lagerungsbedingungen Kristallwachstum und in der Folge eine verstärkte Sedimentation beobachtet, die zu schwer resuspendierbaren Feststoffablagerungen am Boden des Vorratsgefäßes führt, wodurch die Qualität und Dosierungsgenauigkeit dieser Präparate beeinträchtigt wird. Ursache für die physikalische Instabilität des Sulfametrol in wässerigen Suspensionen sind nachträgliche Veränderungen in der Kristallstruktur der wasserfreien Sulfametrol-Modifikation, wenn die Präparate einer Temperaturbelastung ausgesetzt oder über einen längeren Zeitraum gelagert werden. Es ist deshalb von großer Bedeutung, eine möglichst stabile Kristallform für die Herstellung von Sulfametrol enthaltenden Arzneiformen zu verwenden.

Es wurde nun gefunden, daß Sulfametrol in eine neue wasserhaltige, kristalline Modifikation übergeführt werden kann, die sich gegenüber dem bekannten Anhydrat des Sulfametrol durch erhöhte Stabilität auszeichnet und für die Herstellung von beständigen pharmazeutischen Präparaten sehr gut geeignet ist.

Gegenstand der Erfindung ist demnach das neue kristalline Halbhydrat des 3-Methoxy-4-(4′-aminobenzolsulfonamido)-1,2,5-thiadiazols (Sulfametrol) der Formel I

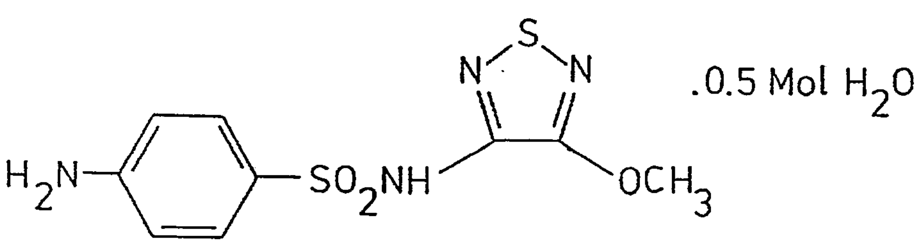

sowie ein Verfahren zu seiner Herstellung, welches dadurch gekennzeichnet ist, daß man wasserfreies, kristallines Sulfametrol mit einer zur guten Durchmischung und Hydratisierung mindestens ausreichenden Wassermenge bei Temperaturen unterhalb von 80°C bis zur Aufnahme des angegebenen Kristallwassergehalts und vollständigen Kristallumwandlung behandelt, die so erhaltenen Kristalle des Sulfametrol-Halbhydrats abtrennt und zur Entfernung vorhandenen Haftwassers bei Temperaturen von 20 bis höchstens 50°C bis zur Gewichtskonstanz trocknet.

Das als Ausgangsmaterial für die Herstellung des erfindungsgemäßen Halbhydrats verwendete wasserfreie, kristalline Sulfametrol kann nach bekannten Verfahren, die beispielsweise in den vorstehend genannten Patentschriften angegeben sind, erhalten werden.

Die Aufnahme des Kristallwassers erfolgt durch Behandeln der wasserfreien Modifikation mit einer zur guten Durchmischung und Bildung des Halbhydrats mindestens ausreichenden Wassermenge. In bevorzugter Weise wird die gewünschte Hydratisierung dadurch erreicht, daß man wasserfreies, kristallines Sulfametrol in reinem Wasser suspendiert, wobei man bis zur Aufnahme des angegebenen Kristallwassergehalts und vollständigen Kristallumwandlung für eine gute Durchmischung der Feststoffanteile mit der zugesetzten Wassermenge sorgt, beispielsweise durch Rühren der Suspension oder Schütteln, Schwenken, Rotieren des Reaktionsgefäßes und dergleichen.

Unter der Bedingung, daß die verwendete Wassermenge zur Bildung eines stöchiometrischen Halbhydrats und zu einer guten Durchmischung der eingesetzten Quantität an Sulfametrol mit Wasser ausreicht, kann man für die Hydratisierung beliebig viel Wasser verwenden, da die Aufnahme von Kristallwasser mit der unter Kristallumwandlung verlaufenden Bildung des Halbhydrats beendet ist und darüber

hinaus keine weitergehende Hydratisierung des Sulfametrol-Halbhydrats stattfindet. Zweckmäßigerweise begrenzt man die Wassermenge jedoch so, daß zwar eine gute Durchmischung stattfinden kann, aber keine oder nur geringe Löslichkeitsverluste auftreten.

Die Hydratisierung wird in bevorzugter Weise bei Raumtemperatur vorgenommen, kann jedoch auch bei erhöhter Temperatur, beispielsweise 40 bis 60° C oder niedriger Temperatur, beispielsweise 5 bis 20° C durchgeführt werden. Die Zeit für die Hydratisierung und Kristallumwandlung hängt insbesondere von der Teilchengröße des Ausgangsmaterials, der eingesetzten Menge an Sulfametrol und von äußeren Bedingungen, wie z.B. Wassermenge, Temperatur, Art und Weise der Agitation, Rührgeschwindigkeit etc. ab. Sie kann so beispielsweise zwischen einigen Stunden und mehreren Tagen liegen. Die vollständige Umwandlung der wasserfreien Modifikation in das Halbhydrat des Sulfametrol wird durch Gewichtskontrolle, Thermomikroskopie, spektroskopische oder kristallogrphische Methoden bestimmt.

Durchmesser, Größe und Form der bei der Hydratisierung entstehenden Kristalle des Sulfametrol-Halbhydrats sind auf verschiedene Weise beeinflußbar.

Wenn während der Hydratisierung nur eine schwache Agitation der Feststoffe in Wasser stattfindet, beispielsweise durch langsames Rühren einer dünnflüssigen Suspension oder durch gelegentliches Schütteln bzw. Rotieren des Reaktionsgefäßes, und die gebildeten Kristalle des Halbhydrats vor dem Abtrennen noch über einen längeren Zeitraum, beispielsweise für 24 Stunden, im wässerigen Milieu aufbewahrt werden, erhält man Kristalle von würfeliger bis rhomboedrischer Form, die einen Durchmesser von ca. 20 bis 80 µm aufweisen.

Andererseits kann man wesentlich kleinere Kristalle des Sulfametrol-Halbhydrats beispielsweise dadurch erhalten, daß man eine dickflüssige Suspension ein bis zwei Tage intensiv rührt, beispielsweise mit einem Magnetrührer mit hoher Drehzahl, und die sich als Bodenkörper absetzende Kristallmasse ohne weitere Behandlung sofort abtrennt. Die Bildung von kleineren Kristallen ist in jenen Fällen vorteilhaft, in denen zur Herstellung bestimmter Arzneiformen ein feines, pulverförmiges Material benötigt wird, welches diesfalls ohne das sonst notwendige Vermahlen direkt weiterverarbeitet werden kann.

Das Abtrennen der Kristalle des Sulfametrol-Halbhydrats vom überschüssigen Wasser geschieht nach üblichen Methoden, beispielsweise durch Filtrieren, Abdekantieren, Zentrifugieren und dergleichen. Das Halbhydrat des Sulfametrol ist so stabil, daß das nach dem Abtrennen noch vorhandene Halftwasser durch Trocknen der feuchten Kristallmasse im Vakuum oder durch mehrstündiges Tempern im Trockenschrank bei Temperaturen bis höchstens 50° C entfernt werden kann, ohne daß es durch diese Behandlung zur Desolvation kommt. Vorteilhafterweise trocknet man jedoch die abgetrennten Kristalle des Halbhydrats im Vakuum bei Raumtemperatur bis zur Gewichtskonstanz.

Die erfindungsgemäße Kristallmodifikation des Sulfametrol besitzt ein charakteristisches IR-Spektrum (Fig. 1), welches charakteristische Absorptionsbanden des Kristallwassers im Bereich der O-H-Valenzschwingungen (3600 -3100 $cm^{-1}$ und bei 1643 $cm^{-1}$ zeigt, die bei der wasserfreien Kristallmodifikation fehlen. Das Halbhydrat unterscheidet sich auch in anderen Schwingungsbereichen von der wasserfreien Kristallmodifikaion des Sulfametrol, sodaß auf eine völlig unterschiedliche Anordnung der Moleküle in den Kristallgittern der beiden Modifikationen geschlossen werden kann.

Die Bestimmung des Wassergehalts bestätigt das Vorliegen eines stöchiometrischen Halbhydrats des Sulfametrol. Der in mehreren Proben des Halbhydrats nach der Karl-Fischer-Methode ermittelte Kristallwassergehalt liegt im Bereich von 0.507 bis 0.517 Mol Wasser pro Mol Sulfametrol.

Bei der thermomikroskopischen Untersuchung des erfindungsgemäßen Halbhydrats werden bei üblicher Aufheizungsgeschwindigkeit ab etwa 80° C Umwandlungserscheinungen und bei 88° C Schmelztropfen, die z.T. wieder kristallisieren, beobachtet. Wird das Thermomikroskop schneller aufgeheizt oder das Präparat zwischen 80 und 90° C auf dem Heiztisch gebracht, ist der Schmelzpunkt des Halbhydrats bei 88° C deutlich zu erkennen. Der Schmelzpunkt der umgewandelten, dehydratisierten Kristalle ist dann mit dem Schmelzpunkt der bekannten wasserfreien Kristallmodifikation des Sulfametrol von 147 - 149° C identisch.

Das mittels DSC (Differential Scanning Colorimetry) unter Atmosphärendruck aufgenommene Thermogramm des Halbhydrats (Fig. 2) zeigt in Übereinstimmung mit den thermomikroskopischen Untersuchungen die bei 80° C beginnende Dehydratisierungsreaktion in Form eines breiten endothermen Peaks, der auf den Umbau des Kristallgitters des untersuchten Halbhydrats, die Dissoziation von Sulfametrol und Wasser und die Verdampfungsenthalpie des freigesetzten Kristallwassers hinweist. Die umgewandelten Kristalle ergeben dann bei etwa 150° C einen scharfen endothermen Peak, der die Schmelzenthalpie anzeigt und im Thermogramm der wasserfreien Kristallmodifikation als einziger endothermer Peak an derselben Stelle auftritt.

Die Absorptionsbanden einer Röntgendiffraktometeraufnahme des Sulfametrol-Halbhydrats sind in Tab. 1 angegeben (siehe Beispiel 1)

Die erfindungsgemäße Kristallmodifikation des Sulfametrol zeigt in pharmazeutischen Präparaten bei Lagerung unter Temperaturbelastung im Vergleich zur wasserfreien Kristallmodifikation eine höhere physikalische Stabilität und ist daher für die Herstellung verschiedener Arzneiformen besser geeignet. Die würfelige bis rhomboedrische Kristallform verleiht dem Sulfametrol-Halbhydrat außerdem ausgezeichnete Fließeigenschaften, was bei der Herstellung von pharmazeutischen Zubereitungen von großem Vorteil ist.

Gegenstand der Erfindung sind daher auch flüssige und feste pharmazeutische Zubereitungen, die

das erfindungsgemäße Sulfametrol-Halbhydrat enthalten, wie z.B. Suspensionen, Emulsionen, Tabletten, Dragees, Drageekerne, Suppositorien, Hart- oder Weichgelatinekapseln und dergleichen. Besonders bevorzugt enthalten wässerige Suspensionen für die orale Applikation das erfindungsgemäße Halbhydrat.

Das Sulfametrol-Halbhydrat kann in diesen pharmazeutischen Zubereitungen als einziger Wirkstoff verwendet oder mit anderen antibakteriell wirksamen Substanzen, vorzugsweise mit Trimethoprim, kombiniert werden.

Pharmazeutische Zubereitungen können das Sulfametrol-Halbhydrat und gegebenenfalls einen oder mehrere andere Wirkstoffe als solche enthalten oder zusammen mit in der Galenik üblicherweise eingesetzten Hilfs- und Zusatzmitteln, wie Tablettenbindern, Füllstoffen, Konservierungsmitteln, Tablettensprengmitteln, Fließregulierungsmitteln, Weichmachern, Netzmitteln, Dispergiermitteln, Emulgatoren, Lösungsmitteln, Geschmackstoffen und dergleichen zu Darreichungsformen für die orale, parenterale oder rektale Applikation formuliert sein.

Die Herstellung der pharmazeutischen Zubereitungen erfolgt in an sich bekannter Weise, beispielsweise durch Mischen, Einrühren, Suspendieren, Dispergieren, Emulgieren usw. der Wirkstoffe mit bzw. in den pharmazeutischen Hilfsstoffen und Verarbeitung zu pharmazeutisch geeigneten Darreichungsformen für die orale, parenterale oder rektale Applikation.

Die Dosierung kann in einer Einheitsdosis beispielsweise zwischen etwa 200 und 600 mg Wirkstoff liegen, während eine Tagesdosis etwa 0.5 bis 5, vorzugsweise 1 bis 3 g betragen kann.

Die nachfolgenden Beispiele erläutern die Erfindung näher.

Beispiel 1:

Herstellung von kristallinem Sulfametrol-Halbhydrat:

100 g (0.35 Mol) kristallines, wasserfreies 3-Methoxy-4-(4'-aminobenzolsulfonamido)-1,2,5-hiadiazol (Sulfametrol) vom Schmp. 147 - 149° C, welches keinen Trocknungsverlust zeigt, werden in 500 ml destilliertem Wasser suspendiert und 72 Stunden bei Raumtemperatur langsam gerührt. Danach wird auf 4° C abgekühlt, die Suspension 24 Stunden bei dieser Temperatur aufbewahrt und der Bodenkörper durch Filtration abgetrennt. Nach dem Trocknen im Vakuum bei Raumtemperatur für 24 Stunden erhält man 103.1 g (100 % der Theorie) kristallines Sulfametrol-Halbhydrat.
Charakteristische Banden des IR-Spektrums, Preßling (0.7 mg Sulfametrol-halbhydrat auf 250 mg KBr, homogen vermischt und gepreßt):
3555, 3470, 3250, 3070 $cm^{-1}$/OH-Valenzschwingungen)
3420, 3370, 3340 (Schulter) $cm^{-1}$ (assymetrische NH-, symmetische NH-und Sulfonamid NH- Valenzschwingungen)
1643 $cm^{-1}$ $H_2O$

Thermomikroskopie:
ab 80° C Umwandlungserscheinungen
88° C inhomogenes Schmelzen der nicht umgewandelten Kristalle
147 - 149° C Schmelzpunkt des umgewandelten, dehydratisierten Produkts

Wassergehalt (nach Karl-Fischer): 3.2 g (0.177 Mol), entspricht 0.507 Mol Wasser pro Mol Sulfametrol

Habitus: Kristalle mit würfeliger bis rhomboedrischer Form, Durchmesser: ca. 20 bis 80 µm

Tabelle 1

Röntgendiffraktometeraufnahme von Sulfametrol-Halbhydrat

| Peak ($cm^{-1}$) | Absorption | Peak ($cm^{-1}$) | Absorption |
|---|---|---|---|
| 3550.94 | 56.49 | 3483.43 | 50.77 |
| 3464.15 | 60.66 | 3417.85 | 53.92 |
| 3365.78 | 66.65 | 3244.26 | 55.08 |
| 3099.60 | 31.62 | 3066.81 | 31.01 |
| 3016.66 | 28.06 | 2970.37 | 25.89 |
| 2947.22 | 26.59 | | |
| 2895.15 | 29.05 | 2787.13 | 25.06 |
| 2663.69 | 22.43 | 2509.38 | 18.20 |
| 2235.49 | 14.05 | 2181.49 | 13.34 |
| 2034.90 | 11.91 | 2000.18 | 11.75 |
| 1928.81 | 10.66 | 1884.45 | 10.08 |
| 1643.35 | 49.23 | 1629.85 | 41.51 |
| 1593.20 | 78.40 | 1560.41 | 59.48 |
| 1512.19 | 67.62 | 1483.26 | 39.61 |
| 1454.32 | 49.72 | 1350.17 | 33.10 |
| 1321.24 | 68.41 | 1307.73 | 61.00 |
| 1178.50 | 48.33 | 1151.50 | 90.18 |
| 1089.78 | 74.06 | | |
| 1004.91 | 31.51 | 968.26 | 17.10 |
| 916.19 | 47.67 | 889.18 | 47.08 |
| 850.61 | 38.34 | 837.10 | 53.84 |
| 744.52 | 25.37 | 682.80 | 58.51 |
| 657.73 | 33.86 | | |

Beispiel 2:

Herstellung von pulverförmigem, fein kristallinem Sulfametrol-Halbhydrat:

100 g (0.35 Mol) kristallines, wasserfreies Sulfametrol werden in 300 ml Wasser suspendiert, die Suspension wird 48 Stunden mit einem Magnetrührer intensiv gerührt und dann der Bodenkörper der Suspension ohne weitere Behandlung abgetrennt.
Nach dem Trocknen im Vakuum erhält man 103.15 g fein kristallines Sulfametrol-Halbhydrat, welches ohne Vermahlen direkt zu festen und flüssigen Arzneiformen weiterverarbeitet werden kann.

Beispiel 3:

**Tablettenformulierung**

| Rezeptur: | 1 Stück | Ansatz für 5000 Tabletten |
|---|---|---|
| Sulfametrol-Halbhydrat | 400 mg | 2000 g |
| Maisstärke | 81.0 mg | 405 g |
| Polyvinylpyrrolidon (mittl. Mol. Gew. 90000) | 11.0 mg | 55 g |
| Magnesiumstearat | 3.0 mg | 15 g |

Verarbeitung:

Sulfametrol-Halbhydrat und Maisstärke werden durch ein Sieb mit Maschenweite 1.00 mm gesiebt, gemischt und mit einer 7%igen wässerigen Lösung von Polyvinylpyrrolidon K 90 granuliert. Das Granulat wird über Nacht bei ca. 50° C getrocknet und durch ein Sieb mit einer Maschenweite von 30 mm gesiebt. Das gesiebte Granulat wird mit Magnesiumstearat homogen vermischt und zu biplanaren Tabletten mit einem Tablettengewicht von 495 mg und einem Durchmesser von 11 mm verpreßt.

Beispiel 4:

Suspension für die orale Applikation

| Rezeptur: | 1 Liter der Suspension |
|---|---|
| Sulfametrol-Halbhydrat | 41.26 g |
| Sorbit flüssig 70 % | 357.14 g |
| Natrium Carboxymethylcellulose | 6.0 g |
| Avicel RC 591 (Mikrokristalline Cellulose und Carboxymethyl-cellulose-Natrium Gemisch) | 12.0 g |
| Polyvinylpyrrolidon K 25 (mittl. Mol. Gew. 25000) | 10.0 g |
| Sorbinsäure | 1.0 g |
| Entmineralisiertes Wasser | q.s |

Verarbeitung:

Natrium Carboxymethylcellulose wird mit Avicel RC 591 gemischt und in 70%igen flüssigen Sorbit eingetragen und homogen vermischt (Suspension I). Polyvinylpyrrolidon wird in 120 m gelöst, Sulfametrol-Halbhydrat und Sorbinsäure eingetragen und homogen vermischt. (Suspension II).
Suspension I und Suspension II werden vereinigt, vermischt und mit demineralisiertem Wasser auf ein Volumen von 1 Liter aufgefüllt.

Stabilitätsprüfung:

Eine nach obiger Rezeptur hergestellte Suspension bleibt bei Lagerung des Präparats unter Temperaturbelastung (40° C) homogen und zeigt unter diesen Bedingungen weder Kristallwachstum noch Sedimentationserscheinungen. Im Vergleich dazu enthalten Suspensionen ähnlicher Rezeptur, die anstelle des Sulfametrol-Halbhydrats mit der wasserfreien Kristallmodifikation von Sulfametrol hergestellt wurden, unter den oben angegebenen Lagerungsbedingungen schwer resuspendierbare Feststoffsedimente als Kuchen am Boden der Vorratsgefäße.

Beispiel 5:

Suppositorienformulierung

| Rezeptur | 1 Stück | Ansatz für 1000 Suppositorien |
|---|---|---|
| Sulfametrol-Halbhydrat | 400 mg | 800 g |
| Witepsol (Glycerinester von Mischungen pflanzlicher gesättigter Fettsäuren | 1339 mg | 1339 g |
| Massa estarinum AB | 447 mg | 447 g |
| Tween 60 | 163 mg | 163 g |

Verarbeitung:

Witepsol, Massa estarinum AB und Tween 60 werden bei 40 - 50° C aufgeschmolzen, der Wirkstoff zugegeben und homogen vermischt. Die homogene Mischung wird anschließend in Formen gegossen.

Beispiel 6:

## Sulfametrol-Halbhydrat-Trimethoprim Kombinationspräparat.

### Sirup für die orale Applikation

| Rezeptur: | 1 Liter Sirup |
|---|---|
| Sulfametrol-Halbhydrat | 41.26 g |
| Trimethoprim | 8.00 g |
| Sorbinsäure | 1.00g |
| Glycerin | 100.00 g |
| Carboxymethylcellulose-Natrium (Viskosität 300 - 600 mPS) | 6.00 g |
| Avicel RC 591 | 12.00 g |
| Saccharose | 250.00 g |
| Polyvinylpyrrolidon K 25 (mittl. Mol. Gew. 25000) | 10.00 g |
| Wasser demineralisiert | q.s. |

Verarbeitung:

Sulfametrol-Halbhydrat, Trimethoprim sowie Sorbinsäure werden unter Rühren in Wasser eingetragen und fein verteilt.
Carboxymethylcellulose-Natrium und Avicel RC 591 werden in Glycerin suspendiert sowie die entsprechende Menge Sirup zugegeben. Dieser Lösung wird die Wirkstoffsuspension zugesetzt.
Dann wird mit Wasser auf das Endvolumen aufgefüllt. Die erhaltene Suspension wird mit einem hochtourigen Rührer homogenisiert und vor dem Abfüllen im Vakuum entlüftet.

**Patentansprüche für die Vertragsstaaten: BE, CH, DE, FR, GB, IT, LI, LU, NL, SE**

1. Kristallines Halbhydrat des 3-Methoxy-4-(4′-aminobenzolsulfonamido)-1,2,5-thiadiazols (Sulfametrol) der Formel I

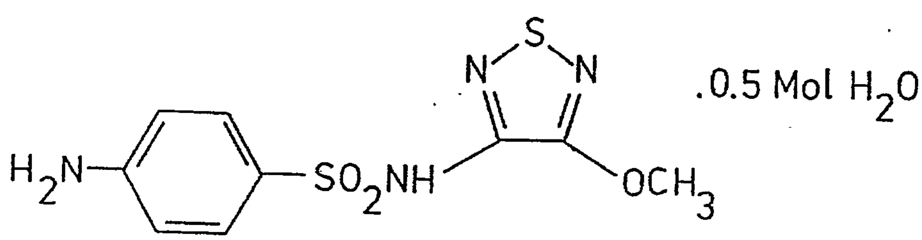

welches im IR-Spektrum einen charakteristischen Peak bei 1643 $cm^{-1}$ besitzt.

2. Verfahren zur Herstellung des kristallinen Halbhydrats der Formel I gemäß Anspruch 1, dadurch gekennzeichnet, daß man wasserfreies, kristallines 3-Methoxy-4-(4′-aminobenzolsulfonamido)-1,2,5-thiadiazol mit einer zur guten Durchmischung und Hydratisierung mindestens ausreichenden Wassermenge bei Temperaturen unterhalb von 80°C bis zur Aufnahme des angegebenen Kristallwassergehalts und vollständigen Kristallumwandlung behandelt, die so erhaltenen Kristalle des Halbhydrats abtrennt

und zur Entfernung vorhandenen Haftwassers bei Temperaturen von 20 bis höchstens 50°C bis zur Gewichtskonstanz trocknet.

3. Verfahren nach Anspruch 2, dadurch gekennzeichnet, daß man eine Suspension des 3-Methoxy-4-(4'-aminobenzolsulfonamido)-1,2,5-thiadiazols in Wasser bis zur vollständigen Hydratisierung und Kristallumwandlung rührt.

4. Verfahren nach den Ansprüchen 2 oder 3, dadurch gekennzeichnet, daß man die Hydratisierung bei Raumtemperatur durchführt.

5. Verfahren nach einem der Ansprüche 2 bis 4, dadurch gekennzeichnet, daß man die erhaltenen Kristalle des Halbhydrats im Vakuum bei Raumtemperatur bis zur Gewichtskonstanz trocknet.

6. Gegen bakterielle Infektionen wirksame pharmazeutische Zubereitungen, dadurch gekennzeichnet, daß sie eine therapeutisch wirksame Menge an kristallinem Halbhydrat der Formel I gemäß Anspruch 1, gegebenenfalls in Kombination mit einer oder mehreren anderen antibakteriell wirksamen Substanzen enthält.

7. Pharmazeutische Zubereitung nach Anspruch 6, dadurch gekennzeichnet, daß sie das kristalline Halbhydrat der Formel I in Kombination mit 2,4-Diamino-5-(3,4,5-trimethoxybenzyl)-pyrimidin enthält.

8. Pharmazeutische Zubereitung nach den Ansprüchen 6 oder 7, dadurch gekennzeichnet, daß sie den oder die Wirkstoffe in Verbindung mit einem oder mehreren in der Galenik üblicherweise verwendeten Hilfs- und Zusatzmitteln enthält.

9. Pharmazeutische Zubereitung nach einem der Ansprüche 6 bis 8 in Form einer stabilen, lagerungsfähigen, wässerigen Suspension für die orale Applikation.

10. Verfahren zur Herstellung von gegen bakterielle Infektionen wirksamen pharmazeutischen Präparaten, dadurch gekennzeichnet, daß das kristalline Halbhydrat der Formel I gemäß Anspruch 1, gegebenenfalls in Kombination mit einer oder mehreren anderen antibakteriell wirksamen Substanzen mit in der Galenik üblichen Hilfsstoffen in eine pharmazeutisch geeignete Darreichungsform für die orale, parenterale oder rektale Applikation gebracht wird.

**Patentansprüche für die Vertragsstaaten: AT, GR, ES**

1. Verfahren zur Herstellung des neuen kristallinen Halbhydrats des 3-Methoxy-4-(4-aminobenzolsulfonamido)-1,2,5-thiadiazols (Sulfametrol) der Formel I

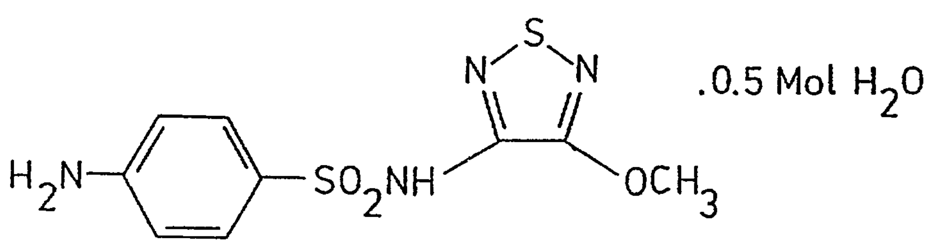

welches im IR-Spektrum einen chakteristischen Peak bei 1643 $cm^{-1}$ besitzt, dadurch gekennzeichnet, daß man wasserfreies, kristallines 3-Methoxy-4-(4-aminobenzolsulfonamido)-1,2,5-thiadiazol mit einer zur guten Durchmischung und Hydratisierung mindestens ausreichenden Wassermenge bei Temperaturen unterhalb von 80°C bis zur Aufnahme des angegebenen Kristallwassergehaltes und vollständigen Kristallumwandlung behandelt, die so erhaltenen Kristalle des Halbhydrats abtrennt und zur Entfernung vorhandenen Haftwassers bei Temperaturen von 20 bis höchstens 50°C bis zur Gewichtskonstanz trocknet.

2. Verfahren nach Anspruch 1, dadurch gekennzeichnet, daß man eine Suspension des 3-Methoxy-4-(4-aminobenzolsulfonamido)-1,2,5-thiadiazols in Wasser bis zur vollständigen Hydratisierung und Kristallumwandlung rührt.

3. Verfahren nach den Ansprüchen 1 oder 2, dadurch gekennzeichnet, daß man die Hydratisierung bei Raumtemperatur durchführt.

4. Verfahren nach einem der Ansprüche 1 bis 3, dadurch gekennzeichnet, daß man die erhaltenen Kristalle des Halbhydrats im Vakuum bei Raumtemperatur bis zur Gewichtskonstanz trocknet.

5. Verfahren zur Herstellung von gegen bakterielle Infektionen wirksamen pharmazeutischen Präparaten, dadurch gekennzeichnet, daß man das kristalline Halbhydrat der Formel I nach Anspruch 1 gegebenenfalls in Kombination mit einem oder mehreren anderen antibakteriellen Wirkstoffen mit in der Galenik üblichen Hilfsstoffen vermischt und in eine pharmazeutische geeignete Darreichungsform für die orale, parenterale oder rektale Applikation bringt.

6. Verfahren nach Anspruch 5 zur Herstellung von pharmazeutischen Präparaten, dadurch gekennzeichnet, daß man eine Wirkstoffkombination bestehend aus dem kristallinen Halbhydrat der Formel I und dem Wirkstoff 2,4-Diamino-5-(3,4,5-trimethoxybenzyl)-pyrimidin in eine pharmazeutisch geeignete Darreichungsform bringt.

7. Verfahren nach einem der Ansprüche 5 oder 6, dadurch gekennzeichnet, daß man das kristalline Halbhydrat der Formel I oder eine dieses enthaltende Wirkstoffkombination mit in der Galenik üblichen Hilfsstoffen vermischt und das Gemisch zu einer stabilen, lagerfähigen, wässrigen Suspension für die orale Applikation verarbeitet.

**Claims for the Contracting States: BE, CH, DE, FR, GB, IT, LI, LU, NL, SE**

1. Crystalline hemihydrate of 3-methoxy-4-(4'-aminobenzenesulfonamido)-1,2,5-thiadiazole (sulfametrole) of the formula I

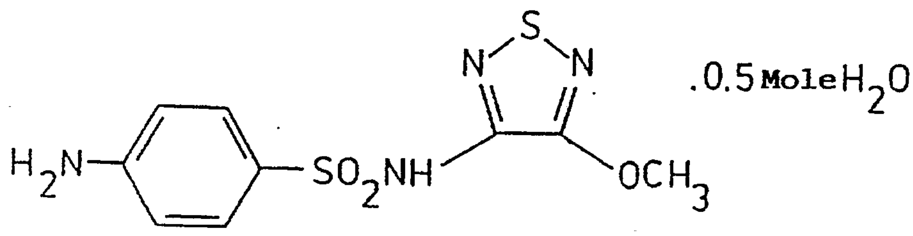

which has a characteristic peak in the IR spectrum at 1643 $cm^{-1}$.

2. Process for the preparation of the crystalline hemihydrate of the formula I according to claim 1, characterized in that anhydrous crystalline 3-methoxy-4-(4'-aminobenzenesulfonamido)-1,2,5-thiadiazole is treated with an amount of water which is at least sufficient for thorough mixing and hydration, at temperatures below 80°C, until the specified content of water of crystallization has been taken up and crystal transformation is complete, and the crystals of the hemihydrate which have thus been obtained are separated off and, to remove adherent water which is present, dried to constant weight at temperatures from 20 to a maximum of 50°C.

3. Process according to claim 2, characterized in that a suspension of 3-methoxy-4-(4'-aminobenzenesulfonamido)-1,2,5-thiadiazole in water is stirred until hydration and crystal transformation are complete.

4. Process according to claims 2 or 3, characterized in that the hydration is carried out at room temperature.

5. Process according to one of claims 2 to 4, characterized in that the resulting crystals of the hemihydrate are dried to constant weight in vacuo at room temperature.

6. Pharmaceutical compositions which are effective against bacterial infections, characterized in that they contain a therapeutically effective amount of crystalline hemihydrate of the formula I according to claim 1, where appropriate combined with one or more other substances having antibacterial activity.

7. Pharmaceutical composition according to claim 6, characterized in that it contains the crystalline hemihydrate of the formula I combined with 2,4-diamino-5(3,4,5-trimethoxybenzyl) pyrimidine.

8. Pharmaceutical composition according to claims 6 or 7, characterized in that it contains the active compound or compounds combined with one or more auxiliaries and additives customarily used in pharmaceuticals.

9. Pharmaceutical composition according to one of claims 6 to 8 in the form of an aqueous suspension, which is stable and can be stored, for oral administration.

10. Process for the preparation of pharmaceutical products effective against bacterial infections, characterized in that the crystalline hemihydrate of the formula I according to claim 1 is converted, where appropriate in combination with one or more other substances having antibacterial activity, with auxiliaries customary in pharmaceuticals into a pharmaceutically suitable presentation for oral, parenteral or rectal administration.

**Claims for the Contracting States: AT, GR, ES**

1. Process for the preparation of the new crystalline hemi-hydrate of 3-methoxy-4-(4-aminobenzenesulfonamido)-1,2,5-thiadiazole (sulfametrole) of the formula I

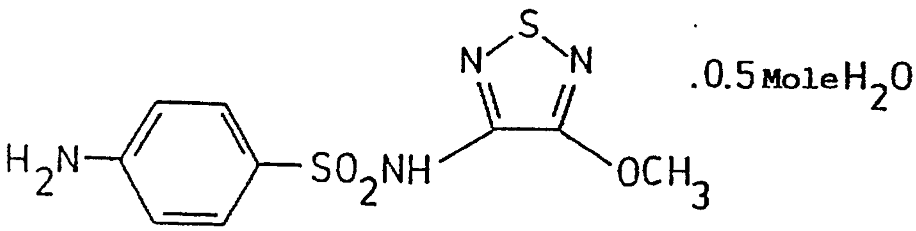

which has a characteristic peak in the IR spectrum at 1643 $cm^{-1}$, characterized in that anhydrous crystalline 3-methoxy-4-(4-aminobenzenesulfonamido)-1,2,5-thiadiazole is treated with an amount of water which is at least sufficient for thorough mixing and hydration, at temperatures below 80°C, until the specified content of water of crystallization has been taken up and crystal transformation is complete, and the crystals of the hemihydrate which have thus been obtained are separated off and, to remove adherent water which is present, dried to constant weight at temperatures from 20 to a maximum of 50°C.

2. Process according to claim 1, characterized in that a suspension of 3-methoxy-4-(4-aminobenzenesulfonamido)-1,2,5-thiadiazole in water is stirred until hydration and crystal transformation are complete.

3. Process according to claims 1 or 2, characterized in that the hydration is carried out at room temperature.

4. Process according to one of claims 1 to 3, characterized in that the resulting crystals of the hemihydrate are dried to constant weight in vacuo at room temperature.

5. Process for the preparation of pharmaceutical products effective against bacterial infections, characterized in that the crystalline hemihydrate of the formula I according to claim 1 is mixed, where appropriate in combination with one or more other compounds having antibacterial activity, with auxiliaries customary in pharmaceuticals and converted into a pharmaceutically suitable presentation for oral, parenteral or rectal administration.

6. Process according to claim 5 for the preparation of pharmaceutical products, characterized in that an active compound combination consisting of the crystalline hemihydrate of the formula I and of the active compound 2,4-diamino-5-(3,4,5-trimethoxybenzyl) pyrimidine is converted into a pharmaceutically suitable presentation.

7. Process according to one of claims 5 or 6, characterized in that the crystalline hemihydrate of the formula I or an active compound combination containing this is mixed with auxiliaries customary in pharmaceuticals, and the mixture is processed to give an aqueous suspension, which is stable and can be stored, for oral administration.

**Revendications pour les Etats contractants: BE, CH, DE, FR, GB, IT, LI, LU, NL, SE**

1. Forme cristalline de semihydrate du 3-methoxy-4(4′-aminobenzolsulfonamido)-1,2,5-thiadiazole (Sulfametrol) de formule I:

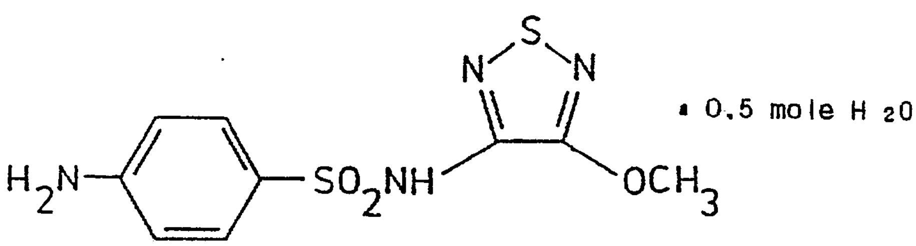

laquelle possède dans son spectre infrarouge un pic caractéristique à 1643 $cm^{-1}$.

2. Procédé de préparation du semihydrate cristallisé de formule I selon la revendication 1, caractérisé en ce qu'on traite le Sulfametrol cristallisé anhydre avec une quantité d'eau suffisante pour un bon mélange et une hydratation à une température inférieure à 80°C jusqu'à l'obtention de la teneur en eau indiquée des cristaux et une transformation cristalline complète, en ce qu'on sépare les cristaux obtenus du semihydrate de Sulfametrol et en ce qu'on sèche jusqu'à masse constante à une température comprise entre 20 et 50°C au plus, pour éliminer l'eau fixée présente.

3. Procédé selon la revendication 2, caractérisé en ce qu'on agite une suspension aqueuse du 3-méthoxy-4(4′-aminobenzolsulfonamido)-1,2,5-thiadiazole jusqu'à hydratation complète et modification cristalline.

4. Procédé selon la revendication 2 ou 3, caractérisé en ce que l'hydratation est réalisée à température ordinaire.

5. Procédé selon l'une des revendications 2 à 4, caractérisé en ce que les cristaux du semihydrate obtenus sont séchés sous vide à température ordinaire jusqu'à masse constante.

6. Préparation pharmaceutique efficace contre les infections bactériennes, caractérisée en ce qu'elle renferme une quantité thérapeutiquement efficace de semihydrate cristallisé de formule I selon la revendication 1, le cas échéant en combinaison avec une ou plusieurs substances antibactériennes.

7. Préparation pharmaceutique selon la revendication 6, caractérisée en ce qu'elle renferme le semihydrate cristallisé de formule I en combinaison avec le 2,4-diamino-5-(3,4,5-triméthoxybenzyl)-pyrimidine.

8. Préparation pharmaceutique selon la revendication 6 ou 7, caractérisée en ce qu'elle renferme la ou les substances actives en combinaison avec un ou plusieurs additifs ou adjuvants utilisés classiquement dans le processus galénique.

9. Préparation pharmaceutique selon l'une des revendications 6 à 8 présentée sous forme de suspension aqueuse apte au stockage et stable pour administration orale.

10. Procédé de préparation de compositions pharmaceutiques efficaces contre les infections bactériennes, caractérisé en ce que le semihydrate cristallisé de formule I selon la revendication 1 est utilisé, le cas échéant en combinaison avec une ou plusieurs substances actives contre les bactéries, avec des additifs chimiques usuels dans le processus galénique, dans une présentation pharmaceutique convenable pour une administration orale, parentérale ou rectale.

**Revendications pour les Etats contractants: AT, ES, GR**

1. Procédé de préparation du nouveau semihydrate cristalliné du 3-méthoxy-4-(4'-aminobenzolsulfonamido)1,2,5-thiadiazole (Sulfametrol) de formule I:

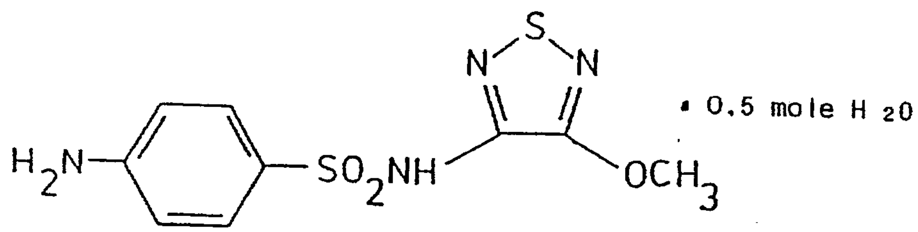

laquelle possède dans son spectre infrarouge un pic caractéristique à 1643 $cm^{-1}$, caractérisé en ce qu'on traite le Sulfametrol cristallisé anhydre avec une quantité d'eau suffisante pour un bon mélange et une hydratation à une température inférieure à 80°C jusqu'à l'obtention de la teneur en eau indiquée des cristaux et une transformation cristalline complète, en ce qu'on sépare les cristaux obtenus du semihydrate de Sulfametrol et en ce qu'on sèche jusqu'à masse constante à une température comprise entre 20 et 50°C au plus, pour éliminer l'eau fixée présente.

2. Procédé selon la revendication 1, caractérisé en ce qu'on agite une suspension aqueuse du 3-méthoxy-4-(4'-aminobenzolsulfonamido)-1,2,5-thiadiazole jusqu'à hydratation complète et modification cristalline.

3. Procédé selon la revendication 1 ou 2, caractérisé en ce que l'hydratation est réalisée à température ordinaire.

4. Procédé selon l'une des revendications 2 à 4, caractérisé en ce que les cristaux du semihydrate obtenus sont séchés sous vide à température ordinaire jusqu'à masse constante.

5. Procédé de préparation de compositions pharmaceutiques efficaces contre les infections bactériennes, caractérisé en ce que le semihydrate cristallisé de formule I selon la revendication 1 est utilisé, le cas échéant en combinaison avec une ou plusieurs substances actives contre les bactéries, avec des additifs chimiques usuels dans le processus galénique, dans une présentation pharmaceutique convenable pour une administration orale, parentérale ou rectale.

6. Procédé selon la revendication 5 de préparation de compositions pharmaceutiques, caractérisé en ce que le semihydrate cristallisé de formule I est utilisé en combinaison avec le substance actif 2,4-diamino-5(3,4,5-trimethoxybenzyl)-pyrimidine dans une présentation pharmaceutique.

7. Procédé selon la revendication 5 ou 6, caractérisé en ce que le semihydrate cristallisé de formule I ou en combinaison actif avec d'autre substances active est agitée avec un ou plusieurs adjuvants utilisés classiquement dans le processus galénique sous forme de suspension apte stockage et stable pour administration orale.

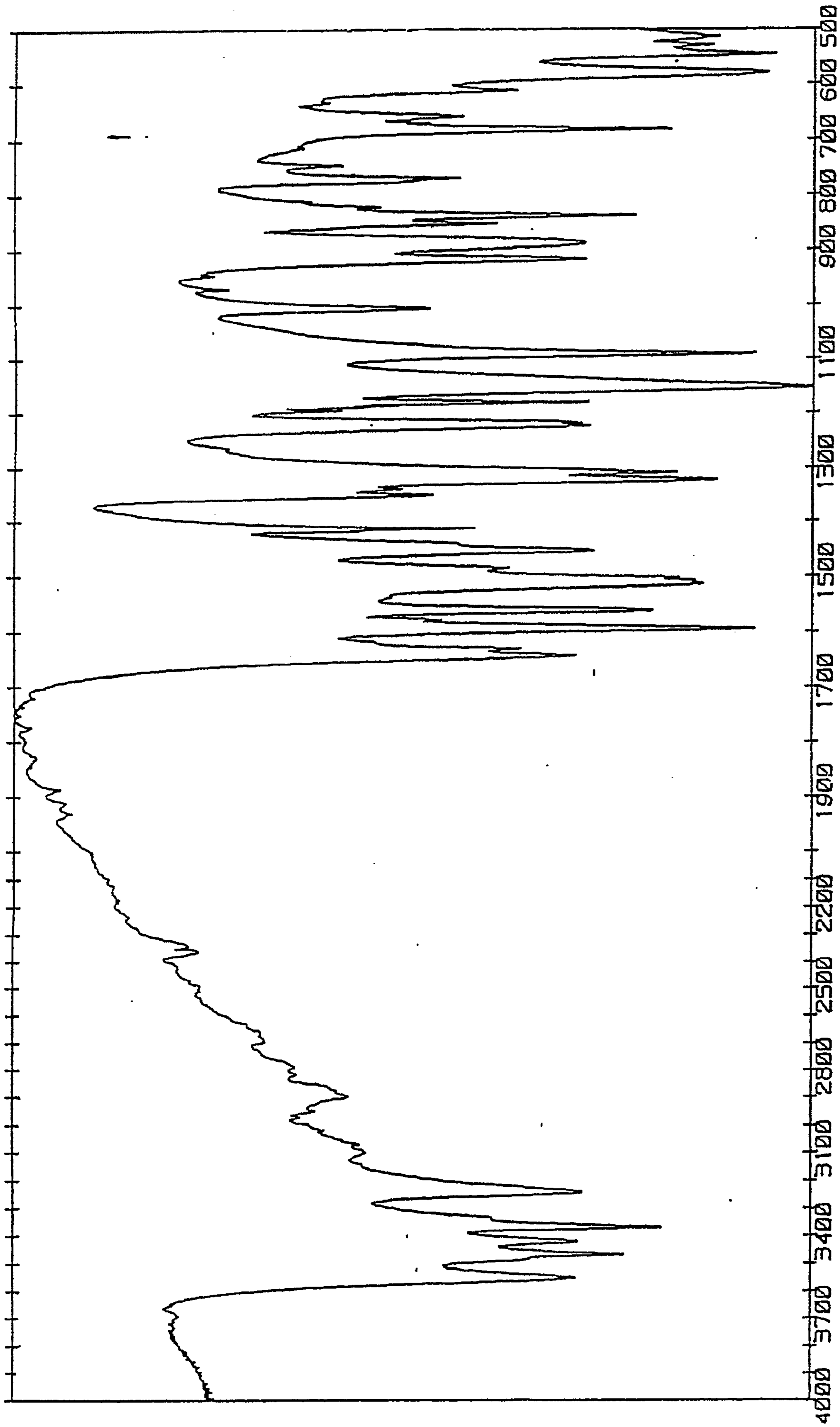
500
600
700
800
900
1100
1300
1500
1700
1900
2200
2500
2800
3100
3400
3700
4000

Fig 1

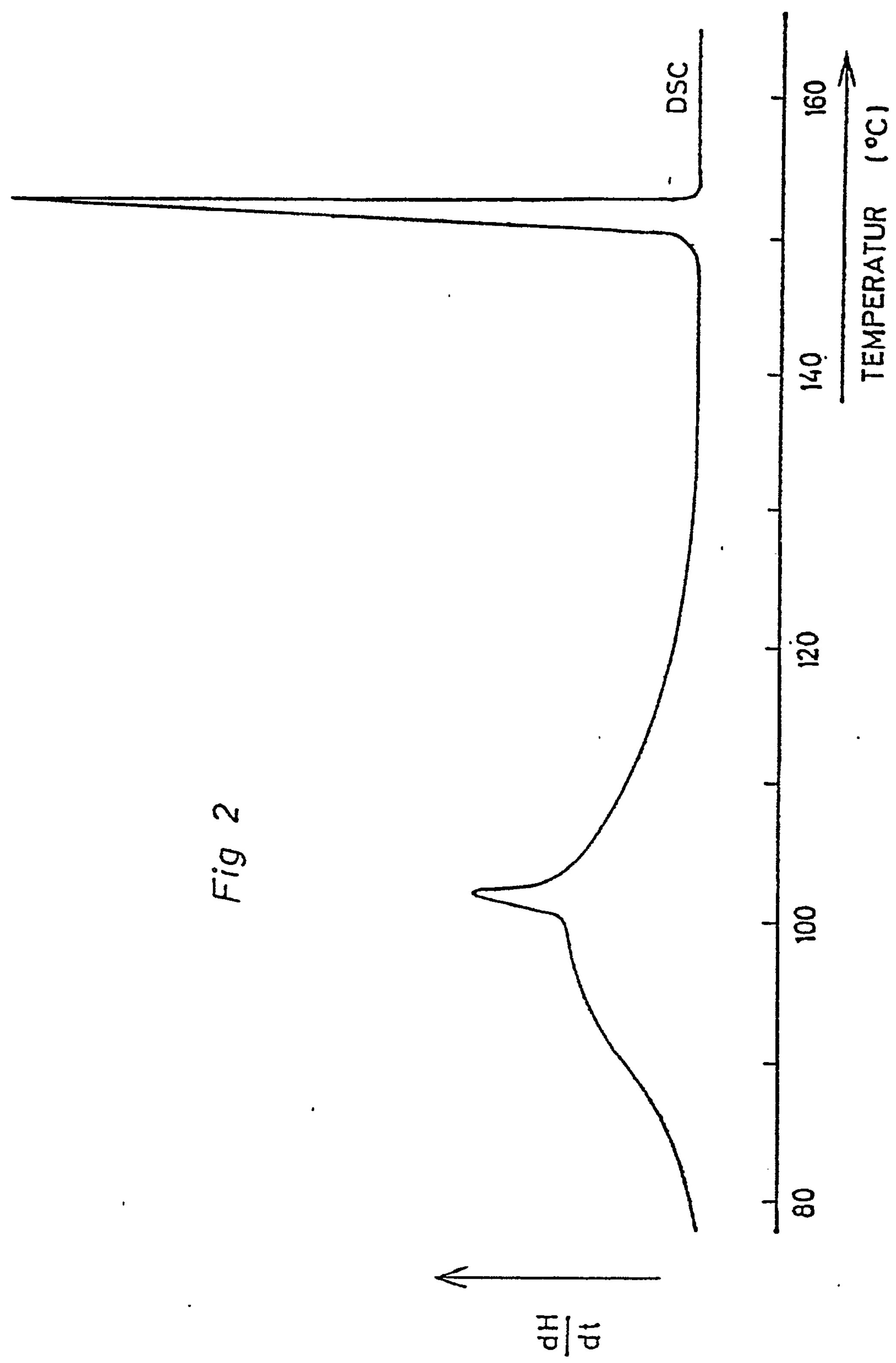


Fig 2